# EUROPEAN PATENT SPECIFICATION

(11) **EP 3 386 466 B1**
(45) Date of publication and mention of the grant of the patent: **11.05.2022**
(21) Application number: 16813204.1
(22) Date of filing: 05.12.2016
(51) Int. Cl.: A61K 31/4045, A61K 31/192, A61P 25/06, A61P 25/24, A61P 25/08, A61P 25/28, A61P 25/16, A61K 8/27, A61K 8/44, A61Q 11/02, A61K 33/00, A61K 31/198

(54) **METAL AMINO ACID COMPLEXES FOR BACTERIAL AGGREGATION**
METALLAMINOSÄUREKOMPLEXE FÜR BAKTERIELLE AGGREGATION
COMPLEXES D'ACIDES AMINÉS-MÉTAL POUR L'AGRÉGATION BACTÉRIENNE

(30) Priority: 07.12.2015 US 201514960960
(43) Date of publication of application: 17.10.2018
(73) Proprietor: Colgate-Palmolive Company, New York, NY 10022 (US)
(72) Inventor: SCHAEFFER-KORBYLO, Lyndsay, Flemington, New Jersey 08822 (US); MANUS, Lisa, Lawrenceville, New Jersey 08648 (US)
(74) Representative: Sonnenhauser, Thomas Martin
(86) International application number: PCT/US2016/064903
(87) International publication number: WO 2017/100116

(56) References cited:
- WO-A1-2015/195124
- US-A1- 2015 306 008
- US-A1- 2015 328 110
- US-A1- 2015 328 111
- US-A1- 2015 328 118
- US-A1- 2015 342 851

## Description

### BACKGROUND

Complexes between metal ions and amino acids are known. Some of these, especially complexes between divalent metal ions and basic amino acids, have seen use in the field of oral care for their ability to treat dentinal hypersensitivity. Certain complexes, such as zinc-bis(lysine)-halide, zinc-bis(arginine)-halide and zinc-(trimethylglycine)halide, have been discovered to form stable, homogenous aqueous solutions, which under certain conditions, can precipitate zinc hydroxide, zinc oxide and other zinc species. The precipitation of such insoluble salts enables oral care compositions comprising these complexes to effectively plug the dentinal tubules of the teeth that transmit sensations of hypersensitivity.

Salts of divalent metal ions, especially zinc, stannous, and copper, are generally known as antibacterial agents. Zinc and stannous salts especially have been used in oral care compositions for their anti-plaque, anti-tarter and anti-malodor effects, which stem from the enhanced bioavailability of ligated zinc ions to interact with oral surfaces. Examples of zinc complexes used in the past include zinc oxide, zinc citrate and zinc chloride. The efficacy and tolerability of these salts have varied due to perceived astringency, cosmetic appearance and interactions between ingredients in these formulations.

The aggregation of bacteria within the human (or other animal) body is an important signal that the immune system uses to promote clearance of harmful bacteria. While individual bacterial cells are difficult for the immune system to detect and clear, larger aggregates are much more efficiently targeted for removal and cleared. Indeed, one important mechanism by which antibodies operate is by causing the aggregation of bacteria, thus flagging them for ingestion by passing phagocytic immune cells. Various molecules, both natural and synthetic, have been described as promoting bacterial aggregation, and can thus exert a beneficial antibacterial effect in the human body.

Free metal ions, including zinc ions, have been demonstrated to promote bacterial aggregation, but this is thought to reflect a non-specific effect on biological membranes. Indeed, this effect has been demonstrated for some eukaryotic cells as well.

While the prior art discloses the use of various metal amino acid complexes in oral compositions, and the use of various metal ions for the aggregation of bacteria, there is still a need for additional compositions and methods which provide selective bacterial aggregation that could be of benefit in oral care compositions. US 2015/0328118 A1 discloses oral care compositions comprising a zinc amino acid halide, methods of making and using the compositions are also provided. US 2015/0328110 A1 discloses dentifrices comprising a zinc amino acid halide, which provide a precipitate of zinc oxide upon use with dilution with water, and/or saliva. US 2015/032811 A1 discloses oral care compositions comprising a tetrabasic zinc halide and an amino acid; along with methods of making and using the same. US 2015/0306008 A1 discloses compositions, e.g., oral and personal care products, comprising (i) a zinc amino acid halide complex, and (ii) cysteine in free or in orally or cosmetically acceptable salt form, together with methods of making and using the same. US 2015/0342851 A1 discloses compositions, e.g., oral and personal care products, comprising (i) a zinc (amino acid or trialkyl glycine) halide complex, and (ii) cysteine in free or in orally or cosmetically acceptable salt form, together with methods of making and using the same. Any reference in the description to methods of treatment refer to the compounds, pharmaceutical compositions and medicaments of the present invention for use in a method of treatment of the human (or animal) body by therapy (or diagnosis).

### BRIEF SUMMARY

It has now been discovered that certain divalent metal ions can form highly soluble complexes with amino acids, and that these complexes can effectively and selectively promote the aggregation of bacteria. Preferred complexes are formed between zinc(II) ions or copper(II) ions and amino acids, especially basic amino acids (e.g., lysine or arginine). Claimed is an oral care composition for use in a method of treating a disease, disorder, or condition of the oral cavity, the method comprising the step of administering to a patient, with *A. viscosus* present in the oral cavity of said patient, an oral care composition comprising a metal amino acid complex, wherein the metal-amino acid complex is a zinc(II)-amino acid complex, selected from zinc-lysine and zinc-arginine complex, by promoting the aggregation and/or immune clearance of said oral bacteria.

In some embodiments, the complex comprising zinc and amino acid as defined in the claims, and optionally an anion and/or oxygen, forms a soluble cationic moiety, which in turn may form a salt with a halide or other anion. While promoting improved bacterial aggregation in comparison to formulations with insoluble zinc salts, the formulations comprising the zinc-amino acid complex do not exhibit the poor taste and mouthfeel, poor fluoride delivery, and poor foaming and cleaning associated with conventional zinc-based oral care products using soluble zinc salts.

In one particular embodiment, the zinc-amino acid complex is a zinc-lysine-HCl complex, for example, the complex designated ZLC, which may be formed from a mixture of zinc oxide and lysine hydrochloride. ZLC has the chemical structure [Zn(C₆H₁₄N₂O₂)₂Cl]⁺ Cl⁻, and may exist in solution of the cationic cation ([Zn(C₆H₁₄N₂O₂)₂Cl]⁺) and the chloride anion, or may be a solid salt, e.g., a crystal, optionally in mono- or dihydrate form. This ZLC complex has been disclosed in WO 2014/098822, WO 2014/098824, WO 2014/098825, and WO 2014/098826.

The oral care compositions for use according to the invention, for example mouthwash, oral gel or dentifrice compositions, comprise a metal-amino acid complex as defined in the claims, e.g. a zinc-amino acid complex, e.g., a zinc - lysine - chloride complex, e.g., ZLC. The compositions may optionally further comprise a fluoride source and or an additional phosphate source. The compositions may be formulated in a suitable oral care formulation e.g., a conventional dentifrice, oral gel or mouthwash base, e.g., comprising one or more abrasives, surfactants, foaming agents, vitamins, polymers, enzymes, humectants, thickeners, antimicrobial agents, preservatives, flavorings, and/or colorants.

Further disclosed are methods of using oral care compositions comprising a metal-amino acid complex, e.g., a zinc-amino acid complex, e.g., a zinc-lysine-chloride complex (ZLC), to promote the aggregation of and immune clearance of oral bacteria. This can result in multiple favorable effects on oral hygiene and oral health, including reduction and inhibition of acid erosion of the enamel, cleaning of the teeth, reduction of bacterially-generated biofilm and plaque, reduction of gingivitis, inhibition of tooth decay and the formation of cavities. The method comprises the application of a composition of the present disclosure to the teeth.

Further areas of applicability of the present invention will become apparent from the detailed description provided hereinafter.

### DETAILED DESCRIPTION

Disclosed is a method of promoting the aggregation and/or immune clearance of oral bacteria, comprising administering to the oral cavity of a person an oral care composition (Composition 1) comprising a metal-amino acid complex. The method can be effective in treating diseases, disorders and conditions of the oral cavity, such as gingivitis, periodontitis, halitosis, cavity formation, enamel erosion, and oral infection (e.g., oral candidiasis), or to disrupt the formation of dental plaque and bacterial biofilm.

The invention refers to an oral care composition as defined in the claims for use in a method of treating a disease, disorder or condition of the oral cavity, comprising the step of administering to a patient in need thereof an oral care composition comprising a metal-amino acid complex as defined in the claims to promote the aggregation and/or immune clearance of oral bacteria. In specific embodiments, said patient suffers from a disease, disorder or condition of the oral cavity, such as gingivitis, periodontitis, halitosis, cavity formation, enamel erosion, and/or oral infection (e.g., oral candidiasis).

The metal-amino acid complex may comprise copper(II) ions.

The compositions may further comprise a halide (e.g., chloride, bromide or fluoride) in ionic association with the metal ion and amino acid.

The molar ratio of metal ion : amino acid may be from 3 : 1 to 1 : 5, e.g., about 1 : 2 and the molar ratio of metal ion : halide where present may be from 3 : 1 to 1 : 3, e.g., about 1 : 2.

The metal ion - amino acid complex may be formed, in whole or in part, *in situ* after the composition is applied.

The metal ion - amino acid complex may be formed, in whole or in part, *in situ* after the composition is formulated.

The amino acid may be lysine.

The metal ion- amino acid complex may be a zinc-lysine complex.

The metal ion (zinc) may be present in an amount of 0.05 to 10% by weight of the composition, optionally at least 0.1, at least 0.2, at least 0.3, at least 0.4, at least 0.5, at least 1, at least 2, at least 3, or at least 4 up to 10% by weight of the composition, e.g. about 1-3%, e.g., about 2-2.7% by weight.

The amino acid may be present in an amount of 0.05 to 30% by weight of the composition, optionally at least 0.1, at least 0.2, at least 0.3, at least 0.4, at least 0.5, at least 1, at least 2, at least 3, at least 4, at least 5, at least 10, at least 15, at least 20 up to 30% by weight, e.g., about 1-10% by weight.

The molar ratio of zinc to amino acid may be 2:1 to 1:4, optionally 1:1 to 1:4, 1:2 to 1:4, 1:3 to 1:4, 2:1 to 1:3, 2:1 to 1:2, or 2:1 to 1:1, e.g., about 1:2 or 1:3

The compositions may comprise a halide in ionic association with zinc and amino acid, wherein the halide is selected from the group consisting of fluorine, chlorine, bromine and mixtures thereof.

The zinc amino acid complex may be a zinc lysine chloride complex (e.g., (ZnLys₂ Cl)⁺Cl⁻ or (ZnLys₃)²⁺Cl₂) or a zinc arginine chloride complex.

The zinc amino acid complex may be a zinc lysine chloride complex, e.g., ZLC, e.g., a zinc lysine chloride complex having the chemical structure [Zn(C₆H₁₄N₂O₂)₂Cl]⁺ Cl⁻, either in solution of the cationic cation (e.g., [Zn(C₆H₁₄N₂O₂)₂Cl]⁺) and the chloride anion, or in solid salt form, e.g., crystal form, optionally in mono- or dihydrate form.

The compositions may be in the form of a toothpaste, gel, mouthwash, powder, cream, strip (e.g., thin films), or gum.

The compositions may be in an orally acceptable base, e.g., a mouthwash, gel, or dentifrice base. The compositions may be in the form of a dentifrice, e.g., wherein the metal - amino acid complex (e.g., zinc-lysine, zinc-arginine) is present in an effective amount, e.g., in an amount of 0.5-4% by weight of metal (e.g., zinc), e.g., about 1-3% by weight of metal (e.g., zinc), in a dentifrice base.

The compositions may be in the form of a dentifrice, wherein the dentifrice base comprises an abrasive, e.g., an effective amount of a silica abrasive, e.g., 10-30%, e.g., about 20%.

The compositions may be in the form of a mouthwash, e.g., wherein the metal - amino acid complex (e.g., zinc-lysine, zinc-arginine) is present in an effective amount, e.g., in an amount of 0.1 to 2% by weight of the composition, or 0.5 to 1.5%, or 1-1.3% by weight of the composition, or wherein the metal - amino acid complex is present in an amount to provide 0.01-0.5% zinc by weight of the composition, e.g., 0.01- 0.25%, or 0.014-0.14% zinc by weight of the composition. The compositions may further comprise an effective amount of a fluoride ion source, e.g., providing 50 to 3000 ppm fluoride.

The compositions may further comprise an effective amount of fluoride, e.g., wherein the fluoride is a salt selected from stannous fluoride, sodium fluoride, potassium fluoride, sodium monofluorophosphate, sodium fluorosilicate, ammonium fluorosilicate, amine fluoride (e.g., N'-octadecyltrimethylendiamine-N,N,N'- tris(2-ethanol)-dihydrofluoride), ammonium fluoride, titanium fluoride, hexafluorosulfate, and combinations thereof.

The compositions may comprise an effective amount of one or more alkali phosphate salts, e.g., sodium, potassium or calcium salts, e.g., selected from alkali dibasic phosphate and alkali pyrophosphate salts, e.g., alkali phosphate salts selected from sodium phosphate dibasic, potassium phosphate dibasic, dicalcium phosphate dihydrate, calcium pyrophosphate, tetrasodium pyrophosphate, tetrapotassium pyrophosphate, sodium tripolyphosphate, and mixtures of any of two or more of these, e.g., in an amount of 1-20%, e.g., 2-8%, e.g., ca. 5%, by weight of the composition.

The compositions may comprise buffering agents, e.g., phosphate buffers or citrate buffers, for example, sodium phosphate buffer (e.g., sodium phosphate monobasic, disodium phosphate and/or phosphoric acid).

The compositions may comprise a humectant, e.g., selected from glycerin, sorbitol, propylene glycol, polyethylene glycol, xylitol, and mixtures thereof, e.g. comprising at least 20%, e.g., 20-40%, e.g., 25-35% glycerin.

The compositions may comprise one or more surfactants, e.g., selected from anionic, cationic, zwitterionic, and nonionic surfactants, and mixtures thereof, e.g., comprising an anionic surfactant, e.g., a surfactant selected from sodium lauryl sulfate, sodium ether lauryl sulfate, and mixtures thereof, e.g. in an amount of from about 0.3% to about 4.5% by weight, e.g. 1-2% sodium lauryl sulfate (SLS); and/or a zwitterionic surfactant, for example a betaine surfactant, for example cocamidopropylbetaine, e.g. in an amount of from about 0.1% to about 4.5% by weight, e.g. 0.5-2% cocamidopropylbetaine.

The compositions may further comprise a viscosity modifying amount of one or more of polysaccharide gums, for example xanthan gum or carrageenan, silica thickener, and combinations thereof.

The compositions may comprise gum strips or fragments.

The compositions may further comprise flavoring, fragrance and/or coloring.

The compositions may comprise an effective amount of one or more antibacterial agents, for example comprising an antibacterial agent selected from halogenated diphenyl ether (e.g. triclosan), herbal extracts and essential oils (e.g., rosemary extract, tea extract, magnolia extract, magnolol, honokiol, thymol, menthol, eucalyptol, geraniol, carvacrol, citral, hinokitol, catechol, methyl salicylate, epigallocatechin gallate, epigallocatechin, gallic acid, miswak extract, sea-buckthorn extract), bisguanide antiseptics (e.g., chlorhexidine, alexidine or octenidine), quaternary ammonium compounds (e.g., cetylpyridinium chloride (CPC), benzalkonium chloride, tetradecylpyridinium chloride (TPC), N-tetradecyl-4-ethylpyridinium chloride (TDEPC)), phenolic antiseptics, hexetidine, octenidine, sanguinarine, povidone iodine, delmopinol, salifluor, metal ions (e.g., zinc salts, for example, zinc citrate, stannous salts, copper salts, iron salts), sanguinarine, propolis and oxygenating agents (e.g., hydrogen peroxide, buffered sodium peroxyborate or peroxycarbonate), phthalic acid and its salts, monoperthalic acid and its salts and esters, ascorbyl stearate, oleoyl sarcosine, alkyl sulfate, dioctyl sulfosuccinate, salicylanilide, domiphen bromide, delmopinol, octapinol and other piperidino derivatives, nicin preparations, chlorite salts; and mixtures of any of the foregoing; e.g., comprising triclosan, or cetylpyridinium chloride or magnolol or honokiol.

The compositions may further comprise a whitening agent, e.g., a selected from the group consisting of peroxides, metal chlorites, perborates, percarbonates, peroxyacids, hypochlorites, and combinations thereof.

The compositions may further comprise hydrogen peroxide or a hydrogen peroxide source, e.g., urea peroxide or a peroxide salt or complex (e.g., such as peroxyphosphate, peroxycarbonate, perborate, peroxysilicate, or persulphate salts; for example calcium peroxyphosphate, sodium perborate, sodium carbonate peroxide, sodium peroxyphosphate, and potassium persulfate);

The compositions may further comprise an agent that interferes with or prevents bacterial attachment, e.g., solbrol or chitosan.

The compositions may further comprise a source of calcium and phosphate selected from (i) calcium-glass complexes, e.g., calcium sodium phosphosilicates, and (ii) calcium-protein complexes, e.g., casein phosphopeptide-amorphous calcium phosphate

The compositions may further comprise a soluble calcium salt, e.g., selected from calcium sulfate, calcium chloride, calcium nitrate, calcium acetate, calcium lactate, and combinations thereof.

The compositions may further comprise a physiologically or orally acceptable potassium salt, e.g., potassium nitrate or potassium chloride, in an amount effective to reduce dentinal sensitivity.

The compositions may further comprise an anionic polymer, e.g., a synthetic anionic polymeric polycarboxylate, e.g., wherein the anionic polymer is selected from 1:4 to 4:1 copolymers of maleic anhydride or acid with another polymerizable ethylenically unsaturated monomer; e.g., wherein the anionic polymer is a methyl vinyl ether/maleic anhydride (PVM/MA) copolymer having an average molecular weight (M.W.) of about 30,000 to about 1,000,000, e.g. about 300,000 to about 800,000, e.g., wherein the anionic polymer is about 1-5%, e.g., about 2%, of the weight of the composition.

The compositions may further comprise a non-ionic polymer, e.g. polyvinylpyrrolidone (PVP), for example linear or cross-linked PVP.

The compositions may further comprise a breath freshener, fragrance or flavoring.

The pH of the composition may be approximately neutral, e.g., from pH 6 to pH 8 e.g., about pH 7.

The compositions may be in the form of a mouthwash wherein the amino acid is lysine and the zinc and lysine form a zinc-lysine-chloride complex having the chemical structure [Zn(C₆H₁₄N₂O₂)₂Cl]⁺ Cl⁻, in an amount to provide 0.5 - 2%, e.g., about 1% zinc by weight, the composition may further comprise humectant, e.g., sorbitol, propylene glycol and mixtures thereof, e.g., in an amount of 10-25%, e.g., about 15-20%, non-ionic surfactant, e.g., poloxamer, e.g., in an amount of 0.1-1%, and sweetener, flavorings, and water, e.g., a mouthwash comprising

| **Ingredients** | **Wt %** |
|---|---|
| Sorbitol | 3-7%, e.g., about 4% |
| ZLC | to provide 0.01-0.5% zinc e.g., 0.01- 0.25%, e.g., about 0.08% |
| Propylene Glycol | 5-10%, e.g., about 7% |
| Surfactant (e.g., Poloxamer, Poloxomer 407, or CAP betaine | 0.1-1%, e.g., about 0.4% |
| Glycerin | 5-10%, e.g., about 7.5% |
| Flavor and/or sweetener | 0.01-1%, e.g., about 0.1% |
| Deionized water | 70-85%, e.g., about 80% |

The compositions may be in the form of an oral gel, wherein the amino acid is lysine and the zinc and lysine form a zinc-lysine-chloride complex having the chemical structure [Zn(C₆Hi₄N₂O₂)₂Cl]^{÷}Cl", in an amount to provide 0.1 - 2%, e.g., about 0.5% zinc by weight, and further comprising humectant, e.g., sorbitol, propylene glycol and mixtures thereof, e.g., in an amount of 45-65%, e.g., about 50-60%, thickeners, e.g., cellulose derivatives, e.g., selected from carboxymethyl cellulose (CMC), trimethyl cellulose (TMC) and mixtures thereof, e.g., in an amount of 0.1-2%, sweetener and/or flavorings, and water, e.g., an oral gel comprising

| **Ingredients** | **Wt %** |
|---|---|
| Sorbitol | 40-60%, e.g., 50-55% |
| ZLC | to provide 0.1-2%Zn, e.g about 0.5% Zn |
| Carboxymethyl cellulose (CMC) and Trimethyl cellulose (TMC) | 0.5-1%, e.g., about 0.7% |
| Flavoring and/or sweetener | 0.01-1% |
| Propylene Glycol | 1-5%, e.g., about 3.00% |

The oral bacteria aggregated by the compositions for use according to the invention include *A. viscous,* and optionally one or more of *L. casei, S. oralis, V. parvula* and F. *nucleatum.* The compositions can be effective to aggregate Gram negative oral bacteria. The patients administered the compositions have demonstrated *A. viscous,* and optionally one or more of *L. casei, S. oralis, V. parvula* and F. *nucleatum* present in their oral cavities. The patients administered the compositions may have been diagnosed with an oral bacterial disease or disorder, e.g., an oral bacterial infection caused by one or more of *A. viscous, L. casei, S. oralis, V. parvula* and F. *nucleatum.* The patients administered the compositions may be suffering from conditions or recovering from treatments that predispose them to oral bacterial infections (e.g., periodontitis, gingivitis, oral surgery, tooth extraction, root canal treatment), i.e. an infection by *A. viscous,* or additionally one or more *of L. casei, S. oralis, V. parvula* and F. *nucleatum.*

The compositions for use according to the invention can further provide effectiveness to reduce and inhibit acid erosion of the enamel, clean the teeth, reduce bacterially-generated biofilm and plaque, reduce gingivitis, inhibit tooth decay and formation of cavities, and reduce dentinal hypersensitivity. The compositions may be used in conjunction with a regimen of tooth brushing, such that brushing dislodges oral bacteria from the structures of the oral cavity (e.g., the teeth and gums) and the resulting the present Methods assist in the aggregation and clearance of these dislodged and free-floating bacteria. The compositions may allow for the aggregation and destruction of oral bacteria transiently released from the oral surfaces (e.g., the bacterial biofilm of the teeth) before they can reattach to solid structures in the oral cavity.

In some embodiments, the compositions are for use in a method which is effective for one or more of the following: (i) to reduce hypersensitivity of the teeth, (ii) to reduce plaque accumulation, (iii) to reduce or inhibit demineralization and promote remineralization of the teeth, (iv) to inhibit microbial biofilm formation in the oral cavity, (v) to reduce or inhibit gingivitis, (vi) to promote healing of sores or cuts in the mouth, (vii) reduce levels of acid producing bacteria, (viii) to increase relative levels of non-cariogenic and/or non-plaque forming bacteria, (ix) to reduce or inhibit formation of dental caries, (x) to reduce, repair or inhibit precarious lesions of the enamel, e.g., as detected by quantitative light-induced fluorescence (QLF) or electrical caries measurement (ECM), (xi) to treat, relieve or reduce dry mouth, (xiii) to reduce erosion, (xv) to reduce tartar build-up, and/or treat or prevent halitosis, and/or (xvii) to promote systemic health, including cardiovascular health, e.g., by reducing the potential for systemic infection via the oral tissues, the method comprising applying any composition, as described above to the oral cavity of a person in need thereof, e.g., one or more times per day.

Claimed is an oral care composition for use in a method of treating a disease, disorder, or condition of the oral cavity, the method comprising the step of administering to a patient, with *A. viscosus* present in the oral cavity of said patient, an oral care composition comprising a metal-amino acid complex, wherein the metal-amino acid complex is a zinc(II)-amino acid complex, selected from zinc-lysine and zinc-arginine complex, by promoting the aggregation and/or immune clearance of said oral bacteria.

The metal-amino acid complex may further comprise a halide, preferably a chloride.

The complex may be a zinc lysine chloride complex having the chemical structure [Zn(C₆H₁₄N₂O₂)₂Cl]⁺ Cl⁻, either in solution of the cationic cation (e.g., [Zn(C₆H₁₄N₂O₂)₂Cl]⁺) and the chloride anion, or in solid salt form, optionally in mono- or dihydrate form.

The amount of zinc may be 0.05-4% by weight.

The composition may be in the form of a toothpaste, gel, mouthwash, powder, cream, strip, or gum.

The composition may be in the form of a mouthwash.

The oral care composition as defined in the claims can be in the form of any oral care formulations, including a toothpaste, gel, mouthwash, powder, cream, strip, gum, or any other known in the art. The oral care composition defined in the claims can be a mouthwash.

The complexes may be the zinc-lysine-chloride complexes referred to as ZLC herein, e.g., a zinc lysine chloride complex having the chemical structure [Zn(C₆H₁₄N₂O₂)₂Cl]⁺ Cl⁻, either in solution of the cation (e.g., [Zn(C₆H₁₄N₂O₂)₂Cl]⁺) and the chloride anion, or in solid salt form, e.g., crystal form, optionally in mono- or dihydrate form. Thus, for example, ZLC, e.g., a zinc lysine chloride complex having the chemical structure [Zn(C₆H₁₄N₂O₂)₂Cl]⁺ Cl⁻, can be used.

Metal-amino acid complexes include those described herein throughout, such as, zinc-amino acid complexes, zinc-amino acid-halide complexes, zinc-lysine-halide complexes, zinc-arginine halide complexes, zinc-lysine-chloride complexes and zinc-arginine-chloride complexes. Such complexes may be the zinc-lysine-chloride complexes referred to as ZLC herein, e.g., a zinc lysine chloride complex having the chemical structure [Zn(C₆H₁₄N₂O₂)₂Cl]⁺ Cl⁻, either in solution of the cationic cation (e.g., [Zn(C₆H₁₄N₂O₂)₂Cl]⁺) and the chloride anion, or in solid salt form, e.g., crystal form, optionally in mono- or dihydrate form. Thus, for example, disclosed is the use of ZLC, e.g., a zinc lysine chloride complex having the chemical structure [Zn(C₆H₁₄N₂O₂)₂Cl]⁺ Cl⁻.

The method may further comprise the administration of an antibacterial agent to provide a synergistic increase in antibacterial effect. This synergistic effect may arise from the ability of the metal amino-acid complex to promote bacterial aggregation and biofilm disruption, thus increasing the ability of the antibacterial agent to access the bacterial cells. Any antibacterial agent disclosed herein may be useful for said synergistic effect, including but not limited to, quaternary ammonium compounds (e.g., cetyl pyridinium chloride), halogenated diphenyl ethers (e.g., triclosan), and magnolia extracts (e.g., magnolol, honokiol).

The oral care composition for use according to the invention includes the metal-amino acid complex as defined in the claims. For example, where the metal-amino acid complex is a zinc-amino acid-halide complex, the precursors which can react *in situ* with water to form the zinc amino acid halide complex include: (i) zinc and an amino acid hydrohalide, or (ii) zinc chloride and amino acid, or (iii) a zinc ion source, an amino acid, and a halogen acid, or (iv) combinations of (i), (ii), and/or (iii). The zinc amino acid halide can be prepared at room temperature by mixing the precursors in a solution, such as water. The *in situ* formation provides ease of formulation. The precursors can be used instead of first having to form the zinc amino acid halide.

Examples of amino acids useful in the metal-amino acid complexes of the oral care compositions include, but are not limited to, the common natural amino acids, e.g.: lysine, arginine, histidine, glycine, serine, threonine, asparagine, glutamine, cysteine, selenocysteine, proline, alanine, valine, isoleucine, leucine, methionine, phenylalanine, tyrosine, tryptophan, aspartic acid, and glutamic acid. The amino acid disclosed herein can be a neutral or acidic amino acid, e.g., glycine. The amino acid disclosed herein can be an alkylated amino acid, e.g., a tri-N-alkylated amino acid, such as trimethylglycine.

The ability of the metal-amino acid complexes to promote the aggregation of oral bacteria, e.g., the zinc-amino acid complexes, is most notable when the complex is formed from a basic amino acid. By "basic amino acid" is meant the naturally occurring basic amino acids, such as arginine, lysine, and histidine, as well as any basic amino acid having a carboxyl group and an amino group in the molecule, which is water-soluble and provides an aqueous solution with a pH of about 7 or greater. Accordingly, basic amino acids include, but are not limited to, arginine, lysine, citrulline, ornithine, creatine, histidine, diaminobutanoic acid, diaminopropionic acid, salts thereof or combinations thereof. In the claimed compositions, the amino acid is lysine or arginine.

The halide may be chlorine, bromine, or iodine, most typically chlorine. The acid addition salt of an amino acid and a halogen acid (e.g., HCl, HBr, or HI) is sometimes referred to herein as an amino acid hydrohalide. Thus one example of an amino acid hydrohalide is lysine hydrochloride.

In certain embodiments, the amount of zinc amino acid halide in the oral care composition used in the present disclosure is 0.05 to 10% by weight of the composition. In certain embodiments, precursors, e.g., zinc and amino acid hydrohalide, are present in amounts such that when combined into the zinc amino acid halide, the zinc amino acid halide would be present in an amount of 0.05 to 10 % by weight of the composition. In either of these embodiments, the amount of the zinc amino acid halide can be varied for the desired purpose, such as a dentifrice or a mouthwash. In other embodiments, the amount of the zinc amino acid halide is at least 0.1, at least 0.2, at least 0.3, at least 0.4, at least 0.5, at least 1, at least 2, at least 3, or at least 4 up to 10% by weight of the composition. In other embodiments, the amount of the zinc amino acid halide is less than 9, less than 8, less than 7, less than 6, less than 5, less than 4, less than 3, less than 2, less than 1, less than 0.5 to 0.05 % by weight of the composition. In other embodiments, the amounts are 0.05 to 5%, 0.05 to 4%, 0.05 to 3%, 0.05 to 2%, 0.1 to 5%, 0.1 to 4%, 0.1 to 3%, 0.1 to 2%, 0.5 to 5%, 0.5 to 4%, 0.5 to 3%, or 0.5 to 2% by weight of the composition.

Zinc can be present in the oral care composition in an amount of 0.05 to 10% by weight of the composition. In other embodiments, the amount of zinc is at least 0.1, at least 0.2, at least 0.3, at least 0.4, at least 0.5, at least 1, at least 2, at least 3, or at least 4 up to 10% by weight of the composition. In other embodiments, the amount of the zinc is less than 9, less than 8, less than 7, less than 6, less than 5, less than 4, less than 3, less than 2, less than 1, less than 0.5 to 0.05 % by weight of the composition. In other embodiments, the amounts are 0.05 to 5%, 0.05 to 4%, 0.05 to 3%, 0.05 to 2%, 0.1 to 5%, 0.1 to 4%, 0.1 to 3%, 0.1 to 2%, 0.5 to 5%, 0.5 to 4%, 0.5 to 3%, or 0.5 to 2% by weight of the composition.

In certain embodiments, amino acid hydrohalide is present in the oral care composition for use according to the invention in an amount of 0.05 to 30% by weight. In other embodiments, the amount is at least 0.1, at least 0.2, at least 0.3, at least 0.4, at least 0.5, at least 1, at least 2, at least 3, at least 4, at least 5, at least 10, at least 15, at least 20 up to 30% by weight. In other embodiments, the amount is less than 30, less than 25, less than 20, less than 15, less than 10, less than 5, less than 4, less than 3, less than 2, or less than 1 down to 0.05% by weight of the composition.

Where precursor materials are present in the oral care composition, they are preferably present in molar ratios approximately as required to produce the desired zinc amino acid halide, although an excess of one material or another may be desirable in certain formulations, e.g., to balance pH against other formulation constituents, to provide additional antibacterial zinc, or to provide amino acid buffer. Preferably, however, the amount of halide is limited, as constraining the level of halide somewhat encourages interaction between the zinc and the amino acid.

In some embodiments, the total amount of zinc in the oral care composition for use according to the invention is 0.05 to 8 % by weight of the composition. In other embodiments, the total amount of zinc is at least 0.1, at least 0.2, at least 0.3, at least 0.4, at least 0.5, or at least 1 up to 8% by weight of the composition. In other embodiments, the total amount of zinc in the composition is less than 5, less than 4, less than 3, less than 2, or less than 1 to 0.05% by weight of the composition.

In certain embodiments, a molar ratio of zinc to amino acid in the oral care composition used in the compositions for use according to the invention is at least 2:1. In other embodiments, the molar ratio is at least 1:1, at least 1:2, at least 1:3, at least 1:4, 2:1 to 1:4, 1:1 to 1:4, 1:2 to 1:4, 1:3 to 1:4, 2:1 to 1:3, 2:1 to 1:2, 2:1 to 1:1, or 1:3. Above 1:4, it is expected that the zinc will be totally dissolved.

In certain embodiments, the oral care composition for use according to the invention is anhydrous. By anhydrous, there is less than 5% by weight water, optionally less than 4, less than 3, less than 2, less than 1, less than 0.5, less than 0.1 down to 0% by weight water.

The carrier in the oral care composition for use according to the invention represents all other materials in the composition other than the zinc amino acid halide complex or its precursors. The amount of carrier is then the amount to reach 100% by adding to the weight of the zinc amino acid halide, including any precursors.

*Active Agents:* The oral care composition for use according to the invention may comprise various agents which are active to protect and enhance the strength and integrity of the enamel and tooth structure and/or to reduce bacteria and associated tooth decay and/or gum disease, including or in addition to the zinc - amino acid - halide complexes. Effective concentration of the active ingredients used herein will depend on the particular agent and the delivery system used. It is understood that a toothpaste for example will typically be diluted with water upon use, while a mouth rinse typically will not be. Thus, an effective concentration of active in a toothpaste will ordinarily be 5-15x higher than required for a mouth rinse. The concentration will also depend on the exact salt or polymer selected. For example, where the active agent is provided in salt form, the counterion will affect the weight of the salt, so that if the counterion is heavier, more salt by weight will be required to provide the same concentration of active ion in the final product. Arginine, where present, may be present at levels from, e.g., about 0.1 to about 20 wt % (expressed as weight of free base), e.g., about 1 to about 10 wt % for a consumer toothpaste or about 7 to about 20 wt % for a professional or prescription treatment product. Fluoride where present may be present at levels of, e.g., about 25 to about 25,000 ppm, for example about 750 to about 2,000 ppm for a consumer toothpaste, or about 2,000 to about 25,000 ppm for a professional or prescription treatment product. Levels of antibacterial agents will vary similarly, with levels used in toothpaste being e.g., about 5 to about 15 times greater than used in mouthrinse. For example, a triclosan toothpaste may contain about 0.3 wt % triclosan.

*Fluoride Ion Source:* The oral care composition for use according to the invention may further include one or more fluoride ion sources, e.g., soluble fluoride salts. A wide variety of fluoride ion-yielding materials can be employed as sources of soluble fluoride in the present compositions. Examples of suitable fluoride ion-yielding materials are found in U.S. Pat. No. 3,535,421, to Briner et al.; U.S. Pat. No. 4,885,155, to Parran, Jr. et al. and U.S. Pat. No. 3,678,154, to Widder et al. Representative fluoride ion sources include, but are not limited to, stannous fluoride, sodium fluoride, potassium fluoride, sodium monofluorophosphate, sodium fluorosilicate, ammonium fluorosilicate, amine fluoride, ammonium fluoride, and combinations thereof. The fluoride ion source may include stannous fluoride, sodium fluoride, sodium monofluorophosphate as well as mixtures thereof. The oral care composition for use according to the invention herein may also contain a source of fluoride ions or fluorine-providing ingredient in amounts sufficient to supply about 25 ppm to about 25,000 ppm of fluoride ions, generally at least about 500 ppm, e.g., about 500 to about 2000 ppm, e.g., about 1000 to about 1600 ppm, e.g., about 1450 ppm. The appropriate level of fluoride will depend on the particular application. A toothpaste for general consumer use would typically have about 1000 to about 1500 ppm, with pediatric toothpaste having somewhat less. A dentifrice or coating for professional application could have as much as about 5,000 or even about 25,000 ppm fluoride. Fluoride ion sources may be added to the compositions for use according to the invention herein at a level of about 0.01 wt. % to about 10 wt. % or about 0.03 wt. % to about 5 wt. %, or about 0.1 wt. % to about 1 wt. % by weight of the composition. Weights of fluoride salts to provide the appropriate level of fluoride ion will obviously vary based on the weight of the counterion in the salt.

*Abrasives:* The oral care composition for use according to the invention, e.g. Composition 1 may include silica abrasives, and may comprise additional abrasives, e.g., a calcium phosphate abrasive, e.g., tricalcium phosphate (Ca₃(PO₄)₂), hydroxyapatite (Ca₁₀(PO₄)₆(OH)₂), or dicalcium phosphate dihydrate (CaHPO₄ • 2H₂O, also sometimes referred to herein as DiCal) or calcium pyrophosphate; calcium carbonate abrasive; or abrasives such as sodium metaphosphate, potassium metaphosphate, aluminum silicate, calcined alumina, bentonite or other siliceous materials, or combinations thereof.

Other silica abrasive polishing materials useful herein, as well as the other abrasives, generally have an average particle size ranging between about 0.1 and about 30 microns, about between 5 and about 15 microns. The silica abrasives can be from precipitated silica or silica gels, such as the silica xerogels described in U.S. Pat. No. 3,538,230, to Pader et al. and U.S. Pat. No. 3,862,307, to Digiulio. Particular silica xerogels are marketed under the trade name Syloid^{®} by the W. R. Grace & Co., Davison Chemical Division. The precipitated silica materials include those marketed by the J. M. Huber Corp. under the trade name Zeodent^{®}, including the silica carrying the designation Zeodent 115 and 119. These silica abrasives are described in U.S. Pat. No. 4,340,583, to Wason. Abrasive materials useful in the preparation of the oral care compositions for use according to the invention herein include silica gels and precipitated amorphous silica having an oil absorption value of less than about 100 cc/100 g silica and in the range of about 45 cc/100 g to about 70 cc/100 g silica. Oil absorption values are measured using the ASTA Rub-Out Method D281. The silicas may be colloidal particles having an average particle size of about 3 microns to about 12 microns, and about 5 to about 10 microns. Low oil absorption silica abrasives particularly useful in the preparation of the compositions described herein are marketed under the trade designation Sylodent XWA^{®} by Davison Chemical Division of W.R. Grace & Co., Baltimore, Md. 21203. Sylodent 650 XWA^{®}, a silica hydrogel composed of particles of colloidal silica having a water content of 29% by weight averaging about 7 to about 10 microns in diameter, and an oil absorption of less than about 70 cc/100 g of silica is an example of a low oil absorption silica abrasive useful in the practice of the present disclosure.

*Foaming agents:* The oral care composition for use according to the invention also may include an agent to increase the amount of foam that is produced when the oral cavity is brushed. Illustrative examples of agents that increase the amount of foam include, but are not limited to polyoxyethylene and certain polymers including, but not limited to, alginate polymers. The polyoxyethylene may increase the amount of foam and the thickness of the foam generated by the oral care carrier component of the composition. Polyoxyethylene is also commonly known as polyethylene glycol ("PEG") or polyethylene oxide. The polyoxyethylenes suitable for this composition will have a molecular weight of about 200,000 to about 7,000,000. In one embodiment the molecular weight will be about 600,000 to about 2,000,000 and in another embodiment about 800,000 to about 1,000,000. Polyox^{®} is the trade name for the high molecular weight polyoxyethylene produced by Union Carbide. The polyoxyethylene may be present in an amount of about 1% to about 90%, in one embodiment about 5% to about 50% and in another embodiment about 10% to about 20% by weight of the oral care carrier component of the oral care compositions. Where present, the amount of of foaming agent in the oral care composition (i.e., a single dose) is about 0.01 to about 0.9 % by weight, about 0.05 to about 0.5% by weight, and in another embodiment about 0.1 to about 0.2 % by weight.

*Surfactants:* The oral care composition for use according to the invention may contain anionic surfactants, for example:
i. water-soluble salts of higher fatty acid monoglyceride monosulfates, such as the sodium salt of the monosulfated monoglyceride of hydrogenated coconut oil fatty acids such as sodium N-methyl N-cocoyl taurate, sodium cocomonoglyceride sulfate,
ii. higher alkyl sulfates, such as sodium lauryl sulfate,
iii. higher alkyl-ether sulfates, e.g., of formula CH₃(CH₂)ₘCH₂(OCH₂CH₂)ₙOSO₃X, wherein m is 6-16, e.g., 10, n is 1-6, e.g., 2, 3 or 4, and X is Na or K, for example sodium laureth-2 sulfate (CH₃(CH₂)₁₀CH₂(OCH₂CH₂)₂OSO₃Na).
iv. higher alkyl aryl sulfonates such as sodium dodecyl benzene sulfonate (sodium lauryl benzene sulfonate)
v. higher alkyl sulfoacetates, such as sodium lauryl sulfoacetate (dodecyl sodium sulfoacetate), higher fatty acid esters of 1,2 dihydroxy propane sulfonate, sulfocolaurate (N-2-ethyl laurate potassium sulfoacetamide) and sodium lauryl sarcosinate.

By "higher alkyl" is meant, e.g., C₆₋₃₀ alkyl. In particular embodiments, the anionic surfactant is selected from sodium lauryl sulfate and sodium ether lauryl sulfate. The anionic surfactant may be present in an amount which is effective, e.g., > 0.01% by weight of the formulation, but not at a concentration which would be irritating to the oral tissue, e.g., <10%, and optimal concentrations depend on the particular formulation and the particular surfactant. For example, concentrations used or a mouthwash are typically on the order of one tenth that used for a toothpaste. In one embodiment, the anionic surfactant is present in a toothpaste at from about 0.3% to about 4.5% by weight, e.g., about 1.5%. The oral care composition for use according to the invention may optionally contain mixtures of surfactants, e.g., comprising anionic surfactants and other surfactants that may be anionic, cationic, zwitterionic or nonionic. Generally, surfactants are those which are reasonably stable throughout a wide pH range. Surfactants are described more fully, for example, in U.S. Pat. No. 3,959,458, to Agricola et al.; U.S. Pat. No. 3,937,807, to Haefele; and U.S. Pat. No. 4,051,234, to Gieske et al. In certain embodiments, the anionic surfactants useful herein include the water-soluble salts of alkyl sulfates having about 10 to about 18 carbon atoms in the alkyl radical and the water-soluble salts of sulfonated monoglycerides of fatty acids having about 10 to about 18 carbon atoms. Sodium lauryl sulfate, sodium lauroyl sarcosinate and sodium coconut monoglyceride sulfonates are examples of anionic surfactants of this type. In a particular embodiment, the compositions for use according to the invention comprise sodium lauryl sulfate.

The surfactant or mixtures of compatible surfactants can be present in the compositions in about 0.1% to about 5.0%, in another embodiment about 0.3% to about 3.0% and in another embodiment about 0.5% to about 2.0% by weight of the total composition.

*Tartar control agents:* In various embodiments, the oral care composition for use according to the invention may comprise an anticalculus (tartar control) agent. Suitable anticalculus agents include without limitation phosphates and polyphosphates (for example pyrophosphates), polyaminopropanesulfonic acid (AMPS), hexametaphosphate salts, zinc citrate trihydrate, polypeptides, polyolefin sulfonates, polyolefin phosphates, diphosphonates. The composition thus may comprise phosphate salts. In particular embodiments, these salts are alkali phosphate salts, i.e., salts of alkali metal hydroxides or alkaline earth hydroxides, for example, sodium, potassium or calcium salts. "Phosphate" as used herein encompasses orally acceptable mono- and polyphosphates, for example, P₁₋₆ phosphates, for example monomeric phosphates such as monobasic, dibasic or tribasic phosphate; dimeric phosphates such as pyrophosphates; and multimeric phosphates, e.g., sodium hexametaphosphate. In particular examples, the selected phosphate is selected from alkali dibasic phosphate and alkali pyrophosphate salts, e.g., selected from sodium phosphate dibasic, potassium phosphate dibasic, dicalcium phosphate dihydrate, calcium pyrophosphate, tetrasodium pyrophosphate, tetrapotassium pyrophosphate, sodium tripolyphosphate, and mixtures of any of two or more of these. In a particular embodiment, for example the compositions comprise a mixture of tetrasodium pyrophosphate (Na₄P₂O₇), calcium pyrophosphate (Ca₂P₂O7), and sodium phosphate dibasic (Na₂HPO₄), e.g., in amounts of ca. 3-4% of the sodium phosphate dibasic and ca. 0.2-1% of each of the pyrophosphates. In another embodiment, the compositions comprise a mixture of tetrasodium pyrophosphate (TSPP) and sodium tripolyphosphate (STPP)( Na₅P₃O₁₀), e.g., in proportions of TSPP at about 1-2% and STPP at about 7% to about 10%. Such phosphates are provided in an amount effective to reduce erosion of the enamel, to aid in cleaning the teeth, and/or to reduce tartar buildup on the teeth, for example in an amount of 2-20%, e.g., ca. 5-15%, by weight of the composition.

*Polymers:* The oral care composition for use according to the invention may also include additional polymers to adjust the viscosity of the formulation or enhance the solubility of other ingredients. Such additional polymers include polyethylene glycols, polysaccharides (e.g., cellulose derivatives, for example carboxymethyl cellulose, or polysaccharide gums, for example xanthan gum or carrageenan gum). Acidic polymers, for example polyacrylate gels, may be provided in the form of their free acids or partially or fully neutralized water soluble alkali metal (e.g., potassium and sodium) or ammonium salts.

Silica thickeners, which form polymeric structures or gels in aqueous media, may be present in the oral care composition for use according to the invention. Note that these silica thickeners are physically and functionally distinct from the particulate silica abrasives also present in the compositions, as the silica thickeners are very finely divided and provide little or no abrasive action. Other thickening agents are carboxyvinyl polymers, carrageenan, hydroxyethyl cellulose and water soluble salts of cellulose ethers such as sodium carboxymethyl cellulose and sodium carboxymethyl hydroxyethyl cellulose. Natural gums such as karaya, gum arabic, and gum tragacanth can also be incorporated. Colloidal magnesium aluminum silicate can also be used as component of the thickening composition to further improve the composition's texture. In certain embodiments, thickening agents in an amount of about 0.5% to about 5.0% by weight of the total composition are used.

The oral care composition for use according to the invention may include an anionic polymer, for example in an amount of from about 0.05 to about 5%. Such agents useful in compositions described herein are disclosed in U.S. Pat. Nos. 5,188,821 and 5,192,531; and include synthetic anionic polymeric polycarboxylates, such as 1:4 to 4:1 copolymers of maleic anhydride or acid with another polymerizable ethylenically unsaturated monomer, preferably methyl vinyl ether/maleic anhydride having a molecular weight (M.W.) of about 30,000 to about 1,000,000, most preferably about 300,000 to about 800,000. These copolymers are available for example as Gantrez. e.g., AN 139 (M.W. 500,000), AN 119 (M.W. 250,000) and preferably S-97 Pharmaceutical Grade (M.W. 700,000) available from ISP Technologies, Inc., Bound Brook, N.J. 08805. The enhancing agents when present are present in amounts ranging from about 0.05 to about 3% by weight. Other operative polymers include those such as the 1:1 copolymers of maleic anhydride with ethyl acrylate, hydroxyethyl methacrylate, N-vinyl-2-pyrollidone, or ethylene, the latter being available for example as Monsanto EMA No. 1103, M.W. 10,000 and EMA Grade 61, and 1:1 copolymers of acrylic acid with methyl or hydroxyethyl methacrylate, methyl or ethyl acrylate, isobutyl vinyl ether or N-vinyl-2-pyrrolidone.

*Water:* The oral care composition for use according to the invention may comprise significant levels of water. Water employed in the preparation of commercial oral compositions should be deionized and free of organic impurities. The amount of water in the compositions includes the free water which is added plus that amount which is introduced with other materials.

*Humectants:* In certain embodiments, it is also desirable to incorporate in the oral care composition for use according to the invention a humectant to prevent the composition from hardening upon exposure to air. Certain humectants can also impart desirable sweetness or flavor to dentifrice compositions. Suitable humectants include edible polyhydric alcohols such as glycerine, sorbitol, xylitol, propylene glycol as well as other polyols and mixtures of these humectants. In some embodiments of the composition described herein, the principal humectant is glycerin, which may be present at levels of greater than 25%, e.g. 25-35% about 30%, with 5% or less of other humectants.

Unless stated otherwise, all percentages of composition components given in this specification are by weight based on a total composition or formulation weight of 100%.

As used throughout, ranges are used as shorthand for describing each and every value that is within the range. Any value within the range can be selected as the terminus of the range.

Unless otherwise specified, all percentages and amounts expressed herein and elsewhere in the specification should be understood to refer to percentages by weight. The amounts given are based on the active weight of the material.

### EXAMPLES

### Example 1

The general reaction for formation of zinc-lysine-chloride complex (ZLC) is as follows:

ZnO + 2(Lysine·HCl) → [Zn(Lysme)₂Cl]Cl·2H₂O (ZLC)

A 2:1 molar ratio of ZnO:Lysine·HCl suspension is prepared with stirring at room temperature for about 12 hours. The mixture is centrifuged. 1ml of supernatant is transferred into an NMR tube. The NMR tube is then placed in a closed test tube filled with ethanol for crystal growth. A number of colorless, cubic crystals are formed after a week. The crystal structure of ZLC crystal is determined by single crystal X-ray diffraction. The dimension of this complex molecule is 1.7nm^{∗}7.8nm^{∗}4.3nm. In this complex, Zn cation is coordinated by two lysine ligands with two N atoms from NH₂ groups and O atoms from carboxylic groups in an equatorial plane. It displays a distorted square-pyramidal geometry with the apical position occupied by a Cl atom. This novel structure gives rise to a positive cation moiety, to which a Cl anion is combined to form an ionic salt.

*Laboratory scale-up synthesis of pure ZLC powder:* 2 mole of Lysine HCl is dissolved in 1000ml DI water with stirring at room temperature, 1 mole of solid ZnO is added slowly to the Lysine HCl solution with stirring and the stirring is continued at room temperature overnight (about 12 hours). The suspension solution is centrifuged at high speed for 15mins. The supernatant is slowly poured into ethanol. A precipitate is formed immediately. Approximately 5-8 ml ethanol is needed to get 1g powder. The ethanol solvent with powder is filtered, and an off-white powder is obtained. The powder is placed in a 50°C oven for drying and an 88% yield of product is obtained. PXRD confirms the purity of ZLC powder compared to ZLC crystal.

*Low-pH preparation of ZLC:* In an alternative method, lysine HCl (2 molar equivalents) is dissolved in deionized water with stirring at room temperature. Concentrated hydrochloric acid equal to approximately 0.5 molar equivalent is added to the reaction mixture. ZnO (1 molar equivalent) is then added to the reaction mixture, and the mixture is stirred until complete dissolution occurs, typically within a minutes to a few hours. The pH of the reaction is kept at 6 or below. The ZLC complex can be kept in solution in the final reaction mixture, which is clear, for more than 3 months at slightly acidic pH (i.e., < pH 7) without precipitation of ZnO. Alternatively, the reaction mixture can be spray dried to yield ZLC powder, or the reaction mixture can be adjusted to a near-neutral pH (e.g., 6.8), and the crystalline salt of ZLC can be isolated upon slow evaporation of the concentrated ZLC reaction mixture.

### Example 2

Presented in Tables 1A and 1B are exemplary mouthwash formulations containing ZLC as the zinc-amino acid complex.

**Table 1A**

| **Mouthwash with ZLC** | | |
|---|---|---|
| **Ingredients** | | **%** |
| Sorbitol 70%sol | | 5.5 |
| ZLC (2.53% Zn) | | 40 |
| Na Saccharin | | 0.02 |
| Propylene Glycol | | 7 |
| Poloxomer 407 | | 0.4 |
| Citric Acid | | 0.02 |
| Potassium Sorbitol | | 0.05 |
| Glycerin | | 7.5 |
| Flavor | | 0.1 |
| Deionized water | | 39.41 |
| Total | | 100 |
| Zn% | | 1.01 |

**Table 1B**

| **Ingredients** | **Weight %** |
|---|---|
| Demineralized Water | 71.38 |
| Cocamidopropyl Betaine | 0.700 |
| Glycerin (99.0-101.0%) | 10.000 |
| Sorbitol | 10.000 |
| Propylene Glycol | 7.000 |
| Sodium Saccharin | 0.020 |
| Potassium Sorbate | 0.050 |
| Flavor | 0.100 |
| CP-4 (Spray Dried Powder) | 0.600 |
| Hydrochloric Acid (35%) | 0.149 |
| ***Total Components*** | 100.000 |
| ***Total Zinc%*** | 0.080 |

### Example 3

An oral gel toothpaste with ZLC as active ingredient is formulated. Zinc ion concentration is about 0.5% (w/w).

**Table 2**

| **Oral gel with ZLC (2.53% Zn)** | |
|---|---|
| **Ingredients** | **%** |
| Sorbitol 70%sol | 76.03 |
| ZLC aqueous solution 2.53%Zn | 20.00 |
| Carboxymethyl cellulose (CMC) and Trimethyl cellulose (TMC) | 0.70 |
| Na Saccharin | 0.27 |
| Propylene Glycol | 3.00 |
| Total | 100.00 |
| %Zn | 0.506 |

### Example 4

Exemplary dentifrice comprising zinc-lysine, 1450 ppm fluoride, and phosphates is prepared as described in Table 3:

**Table 3**

| **Ingredient** | **Wt%** |
|---|---|
| PEG600 | 3 |
| CMC-7 | 0.65 |
| Xanthan | 0.2 |
| Sorbitol | 27 |
| Glycerin | 20 |
| Saccharin | 0.3 |
| Tetrasodium pyrophosphate | 0.5 |
| Calcium pyrophosphate | 0.25 |
| Sodium phosphate dibasic | 3.5 |
| Sodium fluoride (to provide 1450 ppm fluoride) | 0.32 |
| Titanium dioxide | 0.5 |
| Abrasive silica | 8 |
| Thickener silica | 8 |
| ZLC | 7 |
| Sodium lauryl sulfate | 1.5 |
| Flavoring | 1.2 |
| Water | QS |

### Example 5

***Aggregation of Oral Bacteria:*** Stimulated whole saliva from a human volunteer was harvested and mixed either with an untreated control (water), metal salt solutions (0.2% zinc oxide or 0.1% zinc oxide/0.05% zinc citrate mixture), amino acid solutions (0.3% arginine, 1% arginine, or 0.94% lysine), or metal-amino acid complex solutions (1.2% zinc-lysine complex, 1.33% zinc-arginine complex, or 1% copper-lysine complex) as described in the present disclosure. The solutions were incubated for 15 minutes at room temperature in culture dish wells, then photographs were taken. For the metal salts, each dish showed only undissolved and/or precipitated metal salt. For the amino acids, no change was observed compared to the negative control. Large aggregates of material slowly formed and settled to the bottom of the dishes for each metal-amino acid complex well. These results were confirmed both visually and under low-power magnification.

The above described experiment was repeated using round bottom test tubes instead of culture dishes. Photographs of each tube were made at 5-minute intervals for 30 minutes during incubation. These experiments confirmed a time-dependent formation of large aggregates only in the solution containing the metal-amino acid complexes.

To confirm that the aggregation phenomenon observed was due to bacterial aggregation, as opposed to host cell-mediated aggregation, the human saliva sample was first filter-sterilized (0.2 micron vacuum filter) to remove host cells, cellular debris, oral bacteria and large proteins. To this saliva sample was added a 5-species mix of common oral bacteria *(A. viscous, L. casei, S. oralis, V. parvula* and *F. nucleatum).* The same aggregation phenomenon was observed in this experiment as was observed for the whole saliva experiment.

To evaluate the aggregation quantitatively, *Streptococcus oralis* bacteria was suspended in sterile 0.9% NaCl medium in capped culture tubes. To each tube, a different test sample was added, and the absorbance of the medium at 610 nM was monitored over a period of about one hour. Bacteria of this size will remain suspended in solution for several hours before settling out under gravity. This gives such a solution a uniform haziness or opacity that can be quantified by measuring the absorbance of the solution at 610 nm. The aggregation of bacteria results in clumps of bacterial matter, which initially raises absorbance, followed by a sharp drop in absorbance as the bacterial matter settles out, leaving an increasingly transparent solution. Therefore, a decrease in absorbance signals the formation of bacterial aggregates. Results are shown in Table 4 below:

| OD₆₁₀: | 1 minute | 9 minutes | 21 minutes | 28 minutes | 40 minutes | 55 minutes |
|---|---|---|---|---|---|---|
| Bacteria only | 0.514 | 0.530 | 0.513 | 0.512 | 0.512 | 0.505 |
| 1.5% Arginine | 0.492 | 0.473 | 0.455 | 0.449 | 0.441 | 0.428 |
| 5% Arginine | 0.496 | 0.468 | 0.450 | 0.446 | 0.440 | 0.435 |
| 0.94% Lysine | 0.537 | 0.547 | 0.552 | 0.553 | 0.555 | 0.555 |
| 0.35% ZnCl₂ | 0.572 | 0.584 | 0.615 | 0.680 | 0.652 | 0.397 |
| 1.2% Zn-Lys | 0.549 | 0.551 | 0.574 | 0.515 | 0.357 | 0.182 |
| 1.33% Zn-Arg | 0.563 | 0.568 | 0.575 | 0.620 | 0.616 | 0.361 |

The results obtained show that the addition to the culture of a test solution containing no additive, 1.5% arginine, 5% arginine, 0.94% lysine, or 0.35% zinc chloride showed no appreciable change in OD (610 nm) or a rise in OD (610 nm) over the course of the experiment. In contrast, 1.33% zinc-arginine complex as additive results in a brief rise in absorbance which peaks at about 28 minutes, followed by a significant drop in absorbance by about 55 minutes. Similarly, the use of 1.2% zinc-lysine complex results in a peak of absorbance at about 21 minutes, followed by a significant drop by about 55 minutes. These results demonstrate the clear ability of metal-amino acid complexes to promote the rapid aggregation of oral bacteria. Even more pronounced results are obtained using *A. viscous* bacteria, and these are shown in Table 5 below. Only in the case of using zinc-lysine or zinc-arginine complex, the final test result is nearly zero absorbance at 610 nm:

| OD₆₁₀: | 1 minute | 14 minutes | 27 minutes | 46 minutes | 66 minutes |
|---|---|---|---|---|---|
| Bacteria only | 0.494 | 0.495 | 0.488 | 0.484 | 0.472 |
| 1.5% Arginine | 0.438 | 0.432 | 0.427 | 0.418 | 0.424 |
| 5% Arginine | 0.341 | 0.334 | 0.335 | 0.333 | 0.333 |
| 0.94% Lysine | 0.416 | 0.413 | 0.411 | 0.410 | 0.409 |
| 0.35% ZnCl₂ | 0.315 | 0.315 | 0.316 | 0.391 | 0.255 |
| 1.2% Zn-Lysine | 0.454 | 0.554 | 0.175 | 0.032 | 0.014 |
| 1.33% Zn-Arg | 0.436 | 0.474 | 0.623 | 0.369 | 0.025 |

## Claims

1. An oral care composition for use in a method of treating a disease, disorder, or condition of the oral cavity, the method comprising the step of administering to a patient, with *A. viscosus* present in the oral cavity of said patient, an oral care composition comprising a metal amino acid complex, wherein the metal-amino acid complex is a zinc(II)-amino acid complex, selected from zinc-lysine and zinc-arginine complex, by promoting the aggregation and/or immune clearance of said oral bacteria.

2. The oral care composition for use according to claim 1, wherein the metal-amino acid complex further comprises a halide, preferably a chloride.

3. The oral care composition for use according to claim 1, wherein the complex is a zinc-lysine-chloride complex or a zinc-arginine chloride complex, wherein the amount of zinc is 0.05-4% by weight.

4. The oral care composition for use according to claim 1, wherein the complex is a zinc lysine chloride complex having the chemical structure [Zn(C₆H₁₄N₂O₂)₂Cl]⁺Cl⁻, either in solution of the cationic cation [Zn(C₆H₁₄N₂O₂)₂Cl]⁺) and the chloride anion, or in solid salt form, optionally in mono- or dihydrate form.

5. The oral care composition for use according to claim 1, wherein the composition is in the form of a toothpaste, gel, mouthwash, powder, cream, strip, or gum, preferably in the form of a mouthwash.

## Patentansprüche

1. Mundpflegezusammensetzung zur Verwendung in einem Verfahren zur Behandlung einer Krankheit, einer Störung oder eines Zustands der Mundhöhle, wobei das Verfahren den Schritt des Verabreichens einer Mundpflegezusammensetzung, die einen Metall-Aminosäure-Komplex umfasst, wobei der Metall-Aminosäure-Komplex ein Zink(II)-Aminosäure-Komplex ist, ausgewählt aus Zink-Lysin- und Zink-Arginin-Komplex, an einen Patienten, bei dem *A. viscosus* in der Mundhöhle des Patienten vorhanden ist, durch Förderung der Aggregation und/oder der Immunabwehr der oralen Bakterien umfasst.

2. Mundpflegezusammensetzung zur Verwendung nach Anspruch 1, wobei der Metall-Aminosäure-Komplex weiterhin ein Halogenid, vorzugsweise ein Chlorid, umfasst.

3. Mundpflegezusammensetzung zur Verwendung nach Anspruch 1, wobei der Komplex ein Zink-Lysin-Chlorid-Komplex oder ein Zink-Arginin-Chlorid-Komplex ist, wobei die Menge an Zink 0,05-4 Gewichts-% beträgt.

4. Mundpflegezusammensetzung zur Verwendung nach Anspruch 1, wobei der Komplex ein Zink-Lysin-Chlorid-Komplex mit der chemischen Struktur [Zn(C₆H₁₄N₂O₂)₂Cl]⁺Cl⁻ ist, entweder in Lösung des kationischen Kations [Zn(C₆H₁₄N₂O₂)₂Cl]⁺ und des Chloridanions, oder in fester Salzform, wahlweise in Mono- oder Dihydratform.

5. Mundpflegezusammensetzung zur Verwendung gemäß Anspruch 1, wobei die Zusammensetzung in der Form einer Zahnpasta, eines Gels, eines Mundwassers, eines Pulvers, einer Creme, eines Streifens oder eines Kaugummis vorliegt, vorzugsweise in der Form eines Mundwassers.

## Revendications

1. Composition de soins bucco-dentaires pour une utilisation dans une méthode de traitement d'une maladie, d'un trouble ou d'un état de la cavité buccale, la méthode comprenant l'étape d'administration à un patient, avec *A. viscosus* présent dans la cavité buccale dudit patient, d'une composition de soins bucco-dentaires comprenant un complexe métal-acide aminé, dans laquelle le complexe métal-acide aminé est un complexe zinc(II)-acide aminé, choisi parmi le complexe zinc-lysine et zinc-arginine, en favorisant l'agrégation et/ou la clairance immunitaire desdites bactéries buccales.

2. Composition de soins bucco-dentaires pour une utilisation selon la revendication 1, dans laquelle le complexe métal-acide aminé comprend en outre un halogénure, de préférence un chlorure.

3. Composition de soins bucco-dentaires pour une utilisation selon la revendication 1, dans laquelle le complexe est un complexe zinc-lysine-chlorure ou un complexe zinc-arginine-chlorure, la quantité de zinc étant de 0,05 à 4 % en poids.

4. Composition de soins bucco-dentaires pour une utilisation selon la revendication 1, dans laquelle le complexe est un complexe zinc-lysine-chlorure ayant la structure chimique [Zn(C₆H₁₄N₂O₂)₂Cl]⁺Cl⁻, soit en solution du cation cationique [Zn(C₆H₁₄N₂O₂)₂Cl]⁺ et de l'anion chlorure, soit sous forme de sel solide, éventuellement sous forme mono- ou dihydrate.

5. Composition de soins bucco-dentaires pour une utilisation selon la revendication 1, dans laquelle la composition est sous la forme d'un dentifrice, d'un gel, d'un bain de bouche, d'une poudre, d'une crème, d'une bandelette ou d'une gomme, de préférence sous forme la forme d'un bain de bouche.
